# EUROPEAN PATENT SPECIFICATION

(11) **EP 1 000 598 B1**
(45) Date of publication and mention of the grant of the patent: **23.01.2008**
(21) Application number: 99122140.9
(22) Date of filing: 05.11.1999
(51) Int. Cl.: A61F 13/56, A61F 13/62, A44B 18/00

(54) **Connection tape**
Verbindungsband
Bande de connexion

(30) Priority: 10.11.1998 EP 98121337
(43) Date of publication of application: 17.05.2000
(73) Proprietor: MINNESOTA MINING AND MANUFACTURING COMPANY, St. Paul, Minnesota 55133-3427 (US)
(72) Inventor: Selen, Peter, 5993 PG Massbree (NL); Loescher, Claus, D-41468 Neuss (DE); Tesch, Walter, D-41464 Neuss (DE)
(74) Representative: Wilhelm, Stefan

(56) References cited:
- EP-A- 0 148 587
- EP-A- 0 795 307
- GB-A- 2 257 895

## Description

### Field of the Invention

The invention relates to connection tape for the fastening of two articles or two parts of an article to each other. In particular, the invention relates to a fastening system for an absorbent article such as a disposable diaper or disposable garments comprising such connection tape.

### Background of the Invention

There are various fastening systems known for disposable diapers, which generally comprise adhesive tabs to fasten the front and back waist portions of the diaper together when the diaper is positioned on the wearer's body. Generally, the adhesive tabs are firmly attached to at least the rear waist portion of the diaper and an adhesive surface of a fastening tape is adhered to the surface of the front waist portion to form the connection and fit the diaper to the wearer's body.

In GB-A-2 257 895, a diaper is described where the rear waist portion is fastened onto the front waist portion by tape tabs. The tabs include a first section, which is firmly adhered to the rear waist portion of the diaper, and a second section which can be fastened onto the surface of the front waist portion. The first section consists of two strips of tape with adhesive surfaces which are adhered to opposite surfaces of the rear waist portion. The second section of the tape tabs projecting outwardly from the two ends of the waist portion has a hook material bonded to the tape. The hook material is folded back onto itself at the distal end to form a fingerlift portion. The diaper is provided with the tape tabs folded inwardly in inactive storage position. When the diaper is used, the strips are applied to the front waist portion, where a target tape with a surface of loop material is arranged providing a target area for the hook. Thus, a hook and loop connection can be made, which can be opened and closed several times and the strength of which is not effected by baby powder, water or oil. In several embodiments, large target patches are applied on the surface of the front waist portion to allow for adjustability of the fastening system. While adjustability is an important feature of this embodiment, use of a large target patch requires a large amount of loop fabric material, which is expensive for disposable diapers.

In order to minimize the amount of loop material used while preserving a large target area and therefore allowing for adjustability, a further embodiment of GB-A-2 257 895 includes a target patch with loop material on one surface which includes a layer of pressure sensitive adhesive (PSA) on the back surface so that it can be selectively placed on the front waist portion and adhered thereto. In a storage position, the target patch is connected to the fastening strips by a hook and loop connection. When the diaper is used, the target patch is fastened to the front waist band by its adhesive layer. The connection thus made can be reopened by separating the hook and loop connection between the target patch and the fastening strips. The pressure sensitive adhesive of a target patch adheres strongly to the surface of the waist portion, so that the hook and loop connection is separated rather than lifting off the target patch from the surface of the front waist portion. The connection can be refastened or readjusted by fastening the hook surface of the fastening strips to the loop surface of the target patch. In order to achieve a good connection, the hook and loop surfaces need to overlap.

A refastenable tape closure system for disposable diapers comprising a fastening tape, a target tape and a release tape is disclosed in EP 0,148,587. The fastening tape of such closure tape system is adhered through its pressure-sensitive adhesive layer to the backing of the target tape.

In practice, separating the fastening strips from the target patch leads to problems. When the fingerlift section of the fastening strip is peeled away from the surface of the front waist portion, quite often also the target patch adhered to the front waist portion by a layer of strong PSA is lifted. This either leads to separating the target patch from the surface of the front waist portion or, due to the strong bond of the PSA layer, to tearing of the waist portion material.

It is desirable that the fastening system of a diaper comprising a connection tape, allows repeated reopening and reclosing to check the wearer's condition or whether the diaper needs to be changed. It is also desirable to provide a fastening system which cannot only be fastened in one prescribed position but can be adjusted to provide a firm fit of the diaper on the wearer's body.

It is thus the object of the invention to improve the known mechanical fastening system comprising a connection tape in particular such that opening and reclosing operations can be performed much easier with less risk of damaging the absorbent article.

### Summary of the Invention

This object is solved by the connection tape of claim 1 and the fastening system of claim 11. Dependent claims refer to preferred embodiments.

### Detailed Description of the Invention

The connection tape according to the invention comprises a fastening tape having a first section, adapted to be fixed to a first article or a first part of an article and a second section provided with a first fastener member of a two-part mechanical fastener. In case of a diaper, the first section of the fastening tape would be adhered preferably to the back section of the diaper. The second section could have a first fastener member of any known two-part mechanical fastener where the members are capable of being mechanically engaged and mechanically disengaged from each other. Such a mechanical fastener could be, for example, a snap fastener or any other type of fastener known to the skilled person. The preferred type of mechanical fastener is a hook and loop fastener, which can be opened and closed several times and which is not affected by body care articles, i.e. baby powder, oil or the like. This type of fastener can be manufactured inexpensively in large quantities.

The second fastener member of the two-part mechanical fastener (for example the loop fabric corresponding to the hook structure provided on the second section of the fastening tape) is provided on the second surface of the target tape. The first surface of this target tape can be fixed to a second article or a second part of the article, preferably the front waist portion of the diaper. In a preferred embodiment, the first surface of the target tape can be fixed on the front waist portion of the diaper by means of a layer of pressure sensitive adhesive.

The second surface of the target tape, comprising the second fastener member, according to the invention includes a first end section within which the first fastener member cannot be engaged with the second fastener member. This first end section is arranged at the end distal from the first section of the fastening tape when the fastening tape projects from the article or connects the two parts of the article or two articles. In general terms the distal end is the free end of the second section which is distal from the middle area of the fastening tape connecting the first and second portions thereof It preferably includes the free leading edge of the target tape, which faces to the side from which a person would try to grip the second section of the fastening tape when trying to open the mechanical fastener. In other words, the first end section of the target tape is arranged at the user's end of the connection tape and is arranged opposite to the manufacturer's end.

At this first end section, the second fastener member on the second surface of the target tape is shaped so that it cannot be engaged with the first member provided on the fastening tape. In the case of a hook and loop connection, the two members can only be engaged on overlapping surfaces, i.e. where a surface of loop fabric is in contact with the hook structure. Thus, any means of deactivating the members in the area of the end section can be employed to prevent engagement of the members in that area.

It is possible to provide such an area where no fastening elements are present, in many ways. If the fastening members are applied to the target tape by applying, for example, a patch of loop fabric to the tape, this patch of loop fabric can be positioned so that it does not extend until the leading edge. It is also possible to treat the respective member (here: the loop fabric) so that the fastening elements are disabled or removed.

By preventing engagement of the two fastener members in the area of the first end section on the second surface of the target tape, the leading edge of the first surface of the target tape adhered to the surface of the second article or the second part of the article is not affected by peeling forces so that it is not lifted up and peeled away from said surface when the user attempts to separate the two-part mechanical fastener.

This advantage is especially apparent when the two members of the two-part mechanical fastener are laid onto each other than centered so that the first fastener member overlaps the leading edge of the target tape. Gripping the second section of the fastening tape for peeling off from the target tape would result in peeling forces not acting on the leading edge of the target tape but only acting on an area of the target tape spaced from the leading edge, for example in an area shifted to be center of the target tape. Thus, the described problems of the prior art fastener are avoided.

In order to more easily grasp the target tape for separating the same from the release tape in order to apply the target tape to close the diaper, it is advantageous that the target tape at its leading edge is not adhered to the release tape. However, in such a case, the target tape at its leading edge will also not be adhered to the diaper when applied thereto. In order to prevent peeling-off the target tape from the diaper upon lifting the fastening tape from the target tape, there should be a space between the line along which the target tape at its leading edge is adhered to the diaper and the area, in which the first fastener member is able to mechanically engage with the second fastener member of the target tape. Accordingly, in case of providing a fingerlift at the leading edge of the target tape, the area in which the two fastener members can engage each other must be shifted not from the leading edge of the target tape but from the line along which the target tape is adhered to the diaper.

In a preferred embodiment, the second member extends into said first end section of said second surface of said target tape. To prevent engagement of the first and second member, the second member, within said first end section, is covered by a cover tape having an adhesive surface adhered to said second member. This cover tape thus serves to deactivate the second member in the area of the end section simply by covering a part of the second member, thus preventing engagement of the first fastener member, even when it is brought in contact with the end section of the second member. The distance by which the ends of the cover tape on both surfaces of the second fastener member are spaced, is preferably at least 1 mm, more preferably at least 2 mm and especially preferably at least 4 mm.

The cover tape may extend up to the adhesive first surface of the target tape and be adhered thereto so as to provide a fingerlift at said first end section of the target tape. To provide such a fingerlift, the adhesive cover tape can be wrapped around the leading edge of the target tape. The fingerlift thus created serves to provide the user with a handle to grasp the target tape together with the fastening tape when folding the second section of the connection tape outward from the storage position. Therefore, the cover tape is folded around the leading edge of the target tape wherein the area on the first surface of the target tape covered by the cover tape is smaller than the area which is covered by the cover tape on the second surface of the target tape. The cover tape can be folded directly around the leading edge of the target tape or can be folded onto itself prior to contacting the opposite sides of the target tape so as to enlarge the fingerlift.

In an alternative embodiment of the connection tape, the first and second members are provided with mechanically engagable and disengagable fastening elements projecting from said first and second members and wherein said second member within said first end section of said target tape is free of fastening elements. This is an alternative way of providing a first end section according to the invention which cannot be engaged by the first fastener member provided on the fastening tape. In this area, no peel force can result from the user separating the mechanical fastener. Thus, peeling away of the target tape from the outer surface of the diaper and, therefore damaging the diaper is avoided.

The fastening tape, according to a further embodiment, is provided with one adhesive surface on which the first member is mounted and wherein the end portion of said second section of said fastening tape distal from its first section is folded back so as to build a fingerlift. Such a fingerlift makes it easier for the user to separate the two-part mechanical fastener, because the user can pull on the folded back portion of the fastening tape.

Generally, use of pressure sensitive adhesive connecting the various components of the system is preferred. For fixing the first section of the fastening tape to the first part of the article, any type of adhesive with sufficient bond strength and any type of bonding such as ultrasonic welding or thermal bonding can be used. The first surface of the target tape can be provided with a layer of pressure sensitive adhesive, which should provide a bond strength large enough for the target tape to remain on the surface of the second part of the article when the mechanical connection is opened. As already mentioned, the mechanical connection preferably is a hook and loop connection.

It is possible to provide a release tape extending from the fastening tape, the release tape having an adhesive surface and an opposite release surface. The fastening and release tapes are arranged with their adhesive surfaces confronting each other so that they form a Y-shaped structure with the release tape and the second section of the fastening tape being connected to each other and the release tape and the first section of the fastening tape substantially being parallel. In particular, the fastening tape and the release tape are adhered to opposite sides of the first article or the first part of the article. This Y-shaped structure, where the first part of the article (e.g. the back portion of the diaper) is contacted on both its outer and inner surfaces, serves to provide a firm connection to the first article or first part of the article. The use of a release tape as one branch of the Y-shaped structure makes it possible to form a folded back position of the connection tape used as a storage position, in which the target tape with its adhesive surface is positioned against the release surface of the release tape. Thus, the adhesive surface of the target tape is protected during storage so that it cannot be contaminated and does not adhere to other surfaces. In use, it can be easily separated from the release tape and be adhered to the surface of the second article or the second part of the article.

To provide a connection tape of a fastening system of the invention, it could be advantageous to build the fastening tape and release tape as one contiguous tape with one adhesive surface, said tape in a middle section which forms the second section of the fastening tape is folded back so as to form a Z-shaped structure. Especially, in view of the fact that the tape is preferably provided continuously on a roll, a configuration where instead of separating and then reassembling strips of tape one contiguous tape can be used, simplifies manufacture of the connection tape.

According to the invention, it is further provided a roll of tape, wherein the tape is a connection tape tab according to the invention, where said connection tape is planetary wound as a coil or is level-wound. The connection tape consisting of two sections can be prefabricated, i.e. the construction can be built, for example, as explained in the above embodiments. In the manufacture of the diaper, a roll of such tape is provided, from which strips can be cut and applied to the diaper. Because the second section on which also the target tape is positioned, generally is thicker than the first section of the tape, it can be advantageous to provide a roll which is level-wound.

The absorbent article, in particular a disposable diaper, according to the invention comprises a layer of absorbent material for absorbing liquid of a human body, a film layer covering the absorbent layer on the outer surface to prevent the exudes from escaping the diaper, opposite first and second ends of such assembly of layers and a mechanical closure tape for connecting parts of the assembly of layers which each other so as to fasten said assembly of layers on a human body. The mechanical closure tape comprises the connection tape according to the invention with the first section of the fastening tape being mounted to a first part of the film layer and the target tape being adapted to be fixed to a second part of the film layer. On a disposable diaper, the first section of the connection tape can be already adhered to the outside surface of the back portion of the diaper and folded back onto itself so that it is positioned above the inside surface of that back portion in a storage position. In this storage position, preferably, the adhesive layer of the target tape, which is later to be adhered to the second part of the film layer, is in contact with a release surface of the connection tape.

The present invention also provides a fastening system for fastening two parts 15,16 of an article to each other, in particular for fastening of an absorbent article such as a disposable diaper 10 wherein such fastening system comprises a connection tape 22 of the present invention.

### Brief Description of the Drawings

An embodiment of the present invention will now be described in detail with a reference to the drawings, in which like parts are referred to by like numerals. In the drawings,
Fig. 1 shows a perspective view of diaper and a fastening system for attaching to the diaper,
Fig. 2 shows the diaper of Fig. 1 and the fastening system attached thereto,
Figs. 3 and 4 show enlarged views of the areas III and IV of Figs. 1 and 2,
Figs. 5,6,9, and 10 show in sequence perspective views of a diaper, where the fastening system is applied and the connection thus created is reopened, respectively,
Figs. 7,8, and 11 show cross-sectional views taken along the lines VII, VIII, and XI of Figs. 6 and 9,
Fig. 12 to 15 show cross-sectional views similar to those of Figs. 7,8, and 11 and showing the situation in which a fastening system applied for closing the diaper is opened, closed, and reopened again in different closing positions,
Fig. 16 shows an alternative connection tape providing the feature of adhering the connection tape at the diaper for disposal purposes,
Fig. 17 shows a level-wound roll of connection tape,
Fig. 18 shows a planetary-wound roll of connection tape.

### Brief Description of Preferred Embodiments

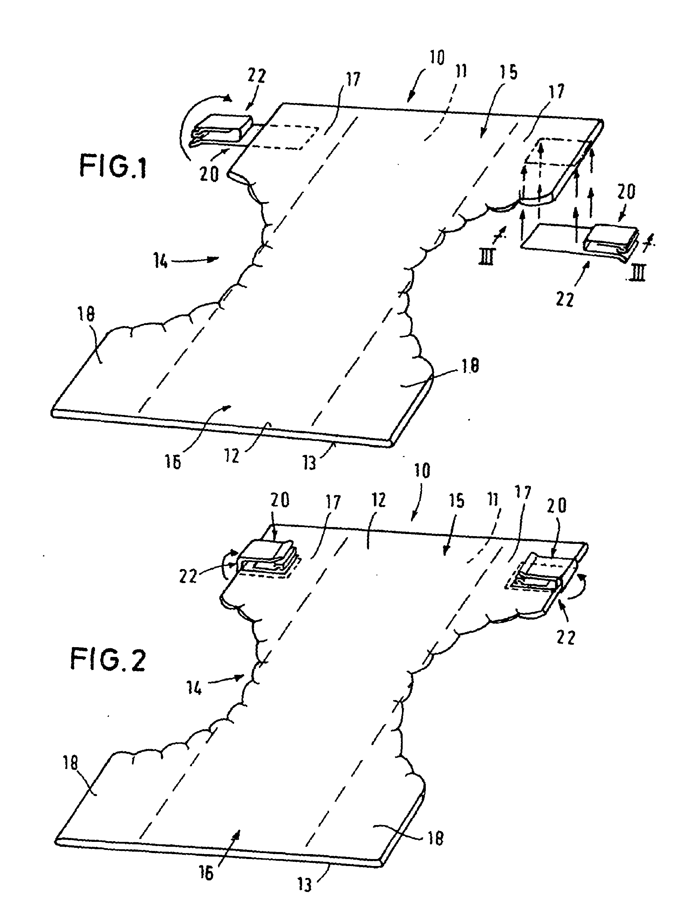

Fig. 1 shows a diaper 10. Diaper 10 can be any type of disposable diaper, composed of an absorbent layer 11 covered on both major surfaces by an inner layer 12 and an outer layer 13. The absorbent layer 11 is made of a non-woven absorbent material. The inner layer 12 is also made from a non-woven material, however is much more thinner and strengthened than the adsorbent layer. Both the inner layer 12 and the absorbent layer 11 are liquid-permeable. The outer layer 13 is made of a liquid-impermeable material such as a polymer material to retain the exudes within the diaper 10. In this description, the further details concerning diaper 10 itself and the material used for the inner, outer, and absorbent layers 12,13,11 will not be specified as such since all of these materials are well known in the art.

Diaper 10 is essentially of an hourglass shape with a narrower intermediate portion 14 arranged between a back waist portion 15 and a front waist portion 16. In use of the diaper, the back waist portion 15 is located at the lower back of the user while the front waist portion 16 is arranged at the belly of the user. Both back and front waist portions 15,16 are provided with lateral back and front ears 17,18 respectively, laterally exceeding beyond the width of the narrower intermediate portion 14. Upon use, the back and front ears 17,18 overlap the back and front waist portions 15,16, respectively, so that these two portions 15,16 build a waist band of the diaper 10 surrounding the waist of the user. As shown in Figs. 1 and 2, each back ear 17 is provided with a fastening system 20 comprising a multiple layer connection tape 22. This connection tape 22 is adhered to both inner and outer layers 12,13 so that each fastening system 20 surrounds the lateral edge of the back ears 17.

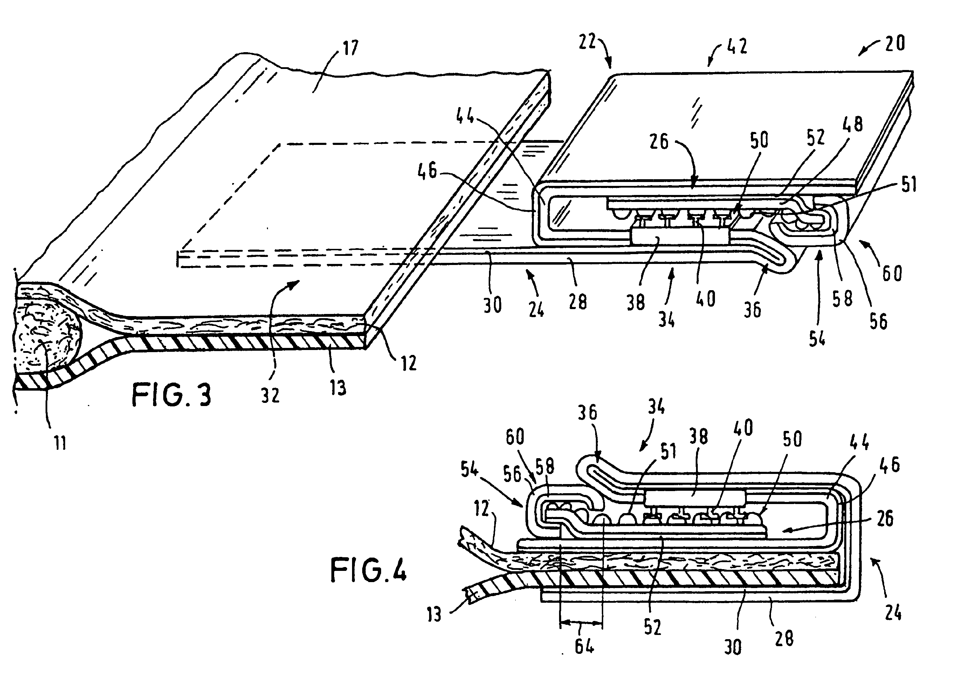

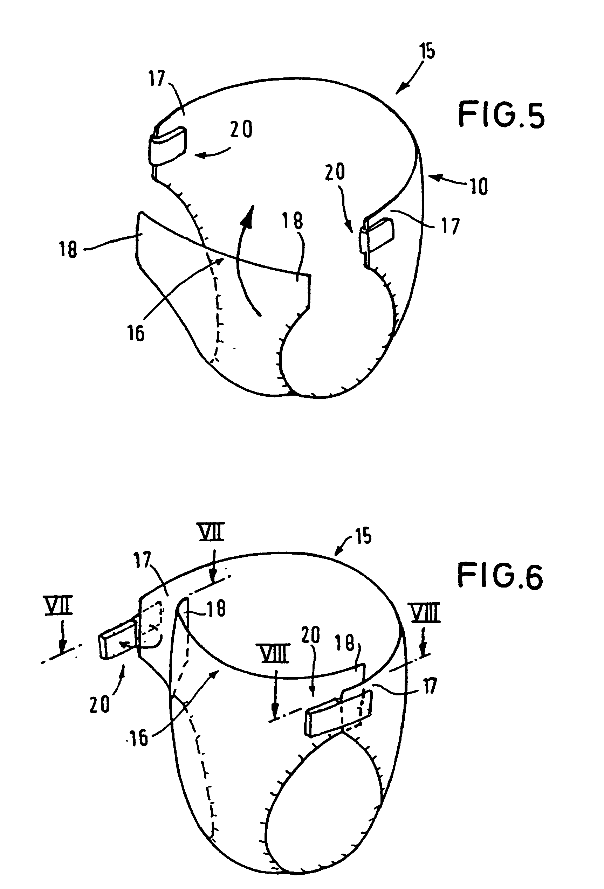

Referring now to Fig. 3, which represents the area marked III of Fig. 1 on a larger scale, there is shown the fastening system with the connection tape 22 in more detail. Connection tape 22 comprises a fastening tape 24 and a target tape 26. The fastening tape 24 comprises a polymeric film backing 28 with a layer 30 of an adhesive. The fastening tape 24 comprises a fixing portion 32 and directly joint thereto a connecting portion 34 in which the fastening tape 24 is folded back onto itself forming a fingerlift 36. The fixing portion 32 is adhered to the outer layer 13 of back ear 17 of the diaper 10 so that the connecting portion 34 projects from the back ear 17 when the fastening tape 24 is in its position to close the diaper 10 (see e.g. Figs. 6,9,11, and 14). On the adhesive layer 30 in the area of the connecting portion 34 of the fastening tape 24, a patch 38 of hook material with projecting hooks 40 is adhered adjacent to the fingerlift 36.

The multiple layer connection tape 22 further comprises a release tape 42 consisting of a backing layer 44 such as a plastic film and an adhesive layer 46 on one surface of the backing layer 44. The release tape 42 is connected to the connecting portion 32 of the fastening tape 24 such that one end of the release tape 42 is arranged adjacent to the patch 38 of hook material with the adhesive layer 46 of the release tape 42 connected to the adhesive layer 30 of the fastening tape 24.

Within the area of the release tape 42, there is arranged the target tape 26 which comprises a backing layer 48 such as a plastic film material with a patch of loop fabric 50 comprising several loops 51 applied to it on one side and an adhesive layer 52 on the opposite side of the backing layer 48. The target tape 26 with its adhesive layer 52 is adhered to the backing layer 44 of the release tape 42 such that in the folded back condition of the release tape 42 (see Fig. 4), the patch of loop fabric 50 and the patch 38 of hook material confront each other. A fingerlift tape 54 in the form of a cover tape comprising a backing layer 56 and an adhesive layer 58 is arranged around the end of the target tape 26 opposite to the end of the release tape 42 connected to the connecting portion 34 of the fastening tape 24. The fingerlift tape 54 covers the patch of loop fabric 50 within the area where the fingerlift tape 54 is applied to the patch of loop fabric 50. Moreover, the fingerlift tape 54 is folded around this end of the patch of loop fabric 50 so that it is adhered to the adhesive layer 52 of the target tape 26 forming a fingerlift 60 at the edge 62 of the target tape.

In Fig. 1, it is shown that connection tape 22 is supplied with fixing portion 32 extended in a line with connecting portion 34. Connection tape 22 is applied to a back ear 17 of the diaper 10 by first applying fixing portion 32 of the fastening tape 24 to the outer layer 13 of diaper 10 (see also Fig. 3) and then folding connecting portion 34 around the edge of the diaper 10 so that the adhesive layer 46 of release tape 42 contacts the inner layer 12 of diaper 10 (Fig. 2). The fixing portion 32 of the fastening tape 24 is sometimes referred to as manufacturer's end and while the connecting portion 34 can be regarded as the user's end.

Referring now to Figs. 5 to 8, it will be explained how the connection tape 22 is used. Fig. 5 shows how the diaper 10 is secured to the body of a wearer, i.e. a baby (not shown), such that the back ears 17 of back waist portion 15 are arranged next to front ears 18 of front waist portion 16.

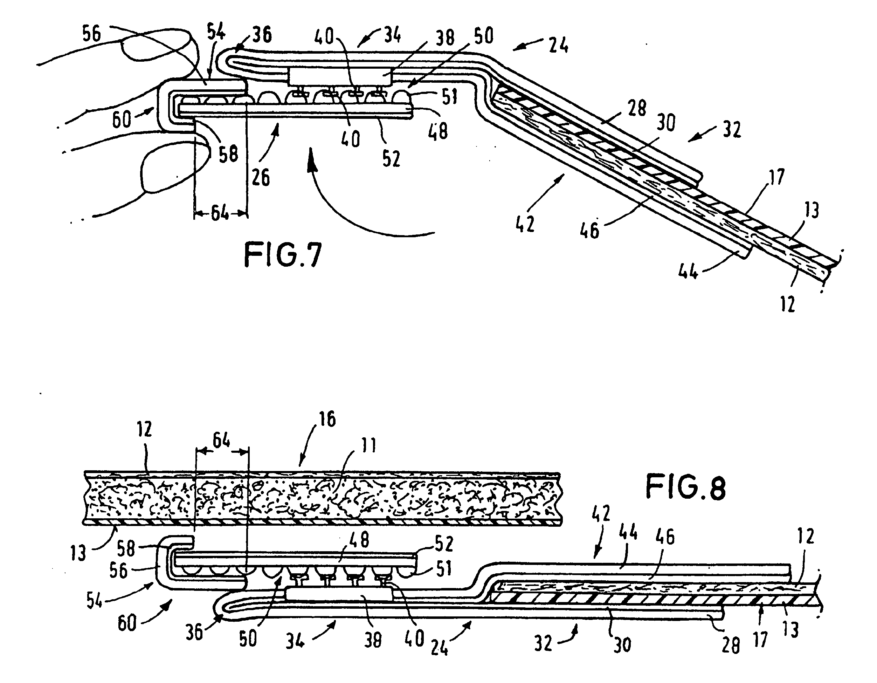

As shown in Figs. 6 and 7, connection tape 22 is folded outwardly, so that fastening tape 24 is straight. The fixing portion 32 of fastening tape 24 remains fixed to back ear 17. Fig. 7 shows in detail how connection tape 22 is folded outwardly, so that target tape 26 is separated from the release tape 42. In order to perform this, the user may grip fingerlift 60 of target tape 26. Fingerlift 60 does not stick to release tape 42 so that the user can grasp this end of target tape 26 easily to peel it off. Target tape 26 remains mechanically connected to the connecting portion 34 of the fastening tape 24 by the hook and loop connection formed by the loops 51 of the patch of loop fabric 50 and the hooks 40 of the patch 38 of hook material, so that by pulling on fingerlift 60 the user folds the connection tape 22 outwardly as shown.

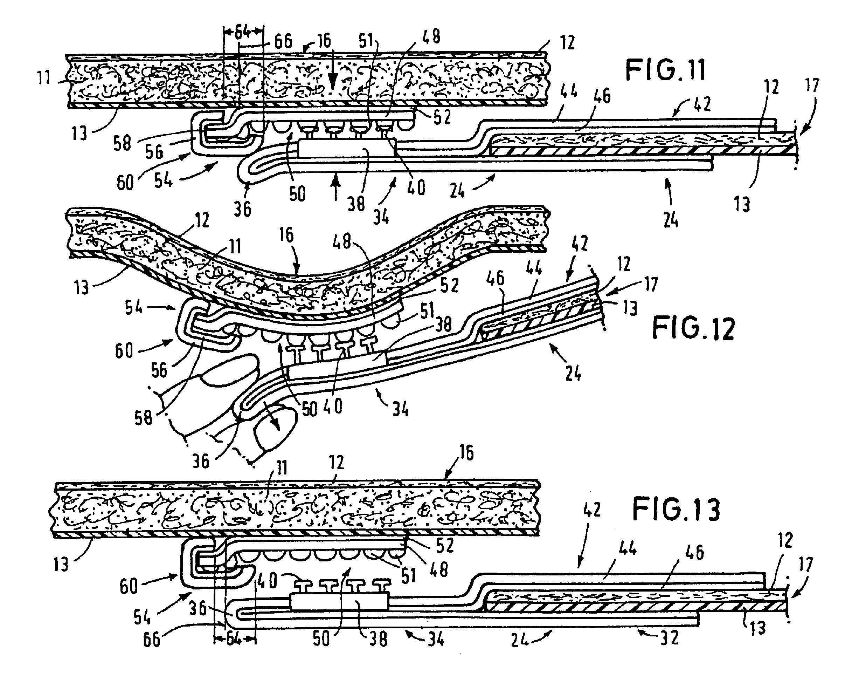

As shown in Figs. 8, 9, and 11, connection tape 22 is fixed to the outer layer 13 of the front waist portion 16 of diaper 10 to connect back ears 17 with front ears 18. Fig. 11 shows how the connection tape 22 contacts the outer layer 13 of the diaper 10. As shown, the adhesive layer 52 of the target tape 26 which is mechanically connected to the connecting portion 34 of the fastening tape 24 is pressed onto the outer layer 13 of the diaper. Adhesive layer 52 sticks to outer layer 13 of diaper 10, so that target tape 26 is firmly fixed to front ear 18. Target tape 26 is still connected to the connecting portion 34 of fastening tape 24 via the hook and loop connection. The fixing portion 32 of fastening tape 24 and the release tape 42 are still firmly fixed to the outer and inner layers 13,12, respectively, of back ear 17 of diaper 10, so that the back and front waist portions 15,16 of diaper 10 are now reliably connected.

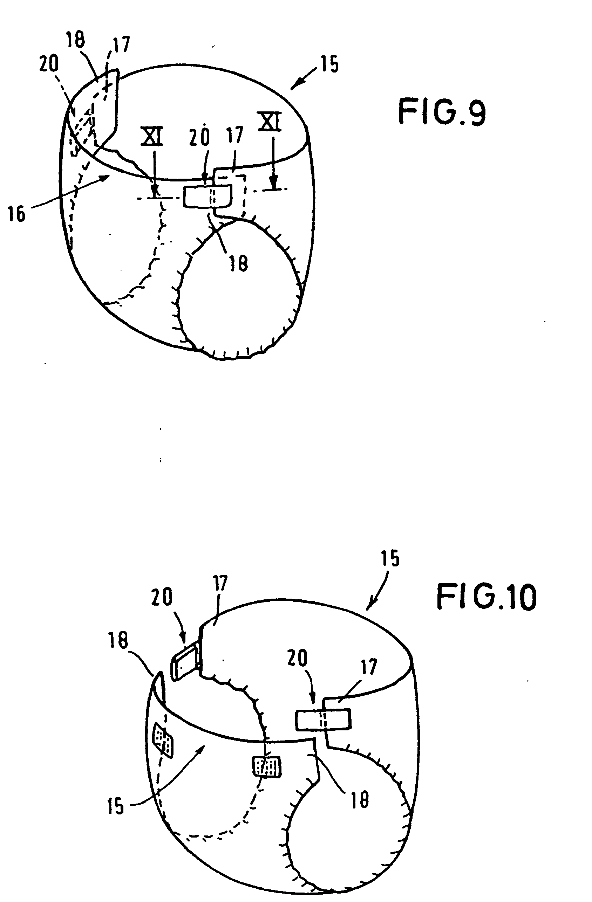

Figs. 10,12, and 13 show how the connection tape 22 can be opened to allow to check the condition of the wearer or to readjust the position of diaper 10.

In Fig. 12, it is shown how the user can separate the connecting portion 34 of the fastening tape 24 from the target tape 26 which is adhered to the outer layer 13 of front waist portion 16. Connecting portion 34 can be separated from target tape 26 by separating the hook and loop connection between loops 51 and hooks 40. This is done by lifting connecting portion 34 from target tape 26. As shown in Fig. 12, the user can grip the overlapping fingerlift 36, which does not carry hooks and therefore does not stick to the loop fabric 50. Thus, the user can apply an upward force to connecting portion 34 to separate the hooks 40 and loops 51.

The strong bond of adhesive layer 52 to outer layer 13 of diaper 10 ensures that target tape 26 will remain fixed to the front waist portion 16 when the connecting portion 34 of the fastening tape 24 is lifted upwardly. Target tape 26 thus remains in place after reopening of connection tape 22, as shown in Fig. 12.

Target tape 26 is not peeled away from outer layer 13 of diaper 10 and also the outer layer 13 is not ruptured as the upward force separating the hook and loop connection is applied. This is due to the fact, that the upward force, which is transmitted over the mechanical hook and loop connection, is well distributed over the area covered by target tape 26. The hooks 40 of hook patch 38 are entangled with loops 51 and contact loop patch 50 only in an inner area of target tape 26 displaced from the fingerlift end of target tape 26 (see fingerlift 60).

While application of hook patch 38 on loop patch 50 in this inner position with no engagement at the fingerlift end of the target tape 26 can be ensured when manufacturing the connection tape 22, this arrangement may differ when the connection is reclosed after opening as discussed below.

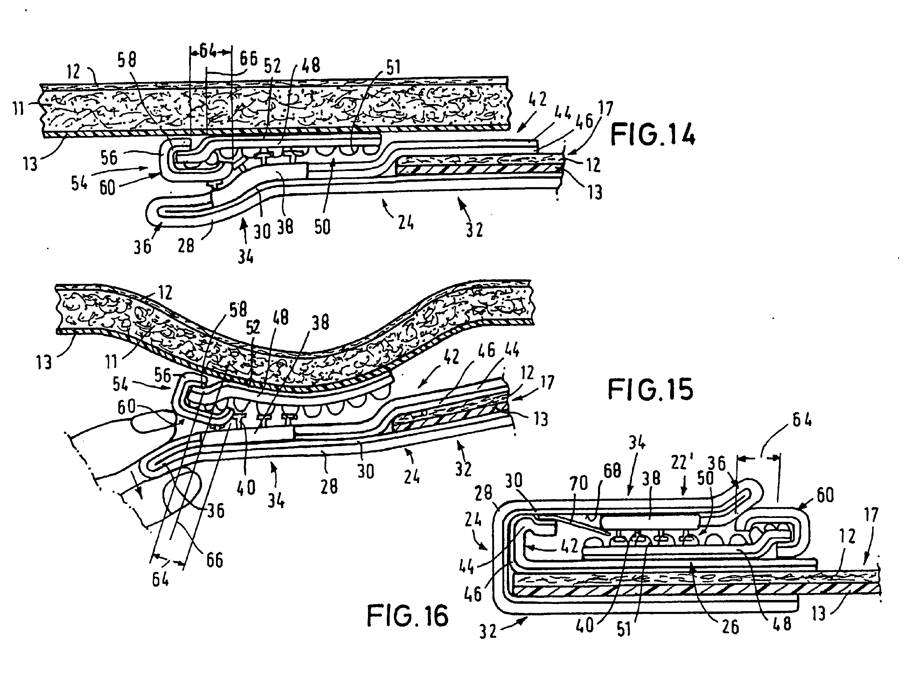

The connection between the back ears 17 and the front ears 18 can be reclosed by bringing the hooks 40 again in contact with the loops 51. If the two patches 38 and 50 are pressed together, they will engage in the area where hook and loop material are in contact. During such operation, it is also possible to secure the connecting portion 34 of fastening tape 24 on target tape 26 not in the centered position shown in Fig. 11 but, for example, in the off-center position of Fig. 14.

As shown in Fig. 14, the connecting portion 34 of fastening tape 24 partially overlaps the edge 62 of target tape 26. Normally, this would lead to problems when fastening tape 24 and target tape 26 are separated by an upward force, because the upward force could lead to peeling off of the target tape 26 from the outer layer 13 or damages of the diaper 10. Such a peeling-off or damage would start along the edge 62 of target tape 26. However, the loops 51 within the area of the edge 62 of target tape 26 are covered by fingerlift tape 54. Thus, in this area, the hooks 40 from hook patch 38 cannot engage the loops 51 of loop fabric 50. Moreover, as shown in the drawings, fingerlift tape 54 covers a larger area over the loops 51 of the loop patch 50 than of the adhesive layer 52. In other words, the fingerlift tape 54, when viewed from the edge 62 of the target tape 26, extends farther over the loop patch 50 than the adhesive layer 52. The difference between the extensions of the fingerlift tape 54 over the loops 51 and over the adhesive layer 52 is referred to in the drawings by reference numeral 64.

In Fig. 15, it is shown how the user separates the connection of Fig. 14 by lifting connecting portion 34 of fastening tape 24 by fingerlift 36. This applies an upward force to connecting portion 34, so that the connection between the hooks and loops 40,51 is separated. Because no hooks and loops are engaged in the area proximate to edge 62 of target tape 26, the upward force is applied in the center of target tape 26 and not along the leading edge 62 or that line 66 along which the target tape at its edge 62 is adhered to the outer layer 13 of the diaper 10. Thus, target tape 26 is not removed from outer layer 13, thereby effectively avoiding any damage to the outside surface layer 12 which would render further reclosure impossible. Accordingly, it is not necessary to hold down the target tape 26 when peeling off the connecting portion 34 of the fastening tape 24 so that the connection tape 22 can be operated by one hand only.

Fig. 16 shows an alternative embodiment for a multiple layer connection tape 22' which is rather similar to that of the preceding Figures. Accordingly, in Fig. 16, those parts of the connection tape 22' which are similar to the parts of the connection tape 22 are provided with the same reference numerals.

In addition to the functions and features of the connection tape 22, the connection tape 22' is provided with an additional adhesion surface within the area of the connecting portion 34 of the fastening tape 24. This additional adhesion surface 68 is shown in Fig. 16 and is located between the patch 38 of hook material and the beginning of the release tape 42. Within this area, the adhesive layer 30 of the fastening tape 24 is not covered by the release tape 42. A release liner 70 covers this adhesion surface 68 and extends to the area between the adhesive layers 30 and 46 of the fastening tape 24 and the release tape 42, respectively. The release liner 70 also extends over the distal edge of patch 38 of hook material.

The additional adhesion surface 68 is useful in order to fix the connecting portion 34 of the fastening tape 24 to the diaper 10 so that the diaper 10 can be closed after use (disposability feature).

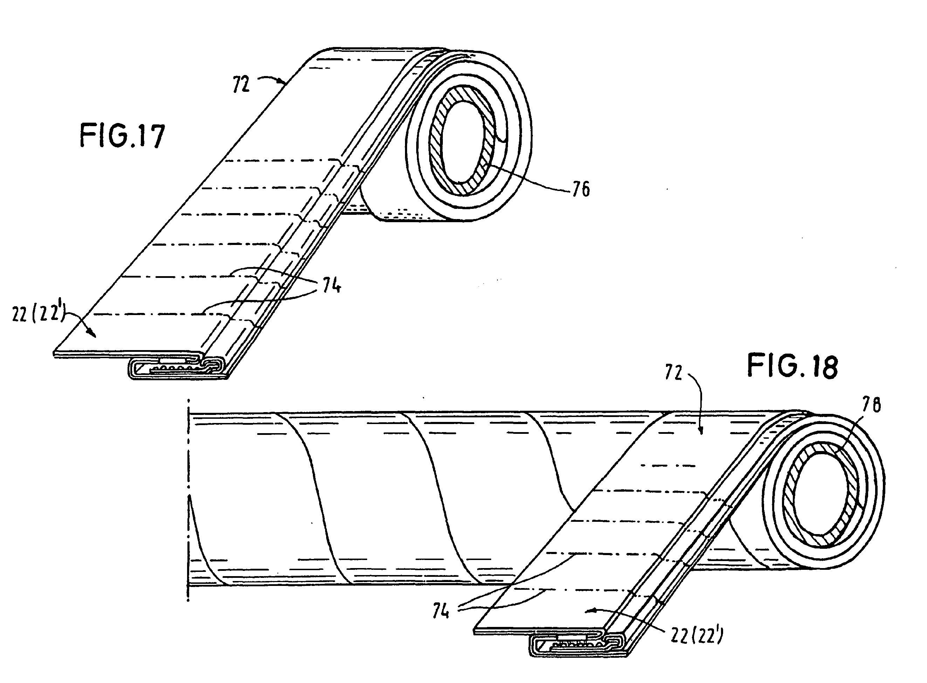

Figs. 17 and 18 show how the multiple layer connection tape is wound on a core for shipping purposes and for storing it in a system to apply the tape to a diaper. The fastening tape, the release tape and the target tape as well as the fingerlift tape are laminated onto each other so as to build a strip 72 of connection tape. By cutting this strip 72 along transverse cutting lines 74, individual multiple layer connection tapes 22,22' can be obtained.

According to Fig. 17, the strip 72 is planetary wound onto a core 76 wherein each layer of the roll comprises only one winding of the strip 72. In contrast thereto, according to Fig. 18, the strip 72 can also be level-wound on a core 78 wherein each layer consists of several windings of the strip 72 adjacent each other.

### Example

In the following, a detailed example of the manufacture of a connection tape 22 is given.

A fastening tape 24 was prepared by coating a 115 µm thick polymeric film based on a cast, white polyethylene-propylene copolymer on one side with a solventless UV-curable silicone low adhesion backsize at a thickness of about 0.5 µm and on the other side with a 35 µm thick coating of synthetic rubber based pressure sensitive adhesive based on a styreneisoprene-styrene block copolymer available as Kraton 1161 from Shell Chemicals. The fastening tape 24 was slit into rolls having a width of 67 mm.

The patch 38 of hook material of a mechanical fastener system bearing hooks on one side having nail-shaped heads was cut into 20 mm wide strips. The hook sheet material had a weight of about 140 g/m² and 388 hooks/mm2. The hook material was obtained as XMH 4156 from 3M Company, St. Paul, Minnesota, USA.

The 20 mm wide hook strip was adhered to the adhesive side of the 67 mm wide fastening tape 24 described above at a distance of 14 mm from one end of the fastening tape. The 14 mm wide strip of fastening tape 24 adjacent to the hook strip was folded over mechanically onto itself (adhesive to adhesive) to form the non-adhesive fingerlift 36 of about 7 mm in width adjacent to the patch 38 of hook material.

A loop tape was prepared by first obtaining a knitted polyester loop material available as Fabric 747 from Milliken (LaGrange, GA, USA). The reverse side of the loop material was thermally bonded to a polyolefin polymer film with a base weight of about 40 g/m². The loop material was then treated on the loop-bearing surface with a low adhesion backsize based on a silicone polyurea. The polymeric film surface was coated with a 38 µm thick layer 52 of pressure sensitive adhesive based on a synthetic rubber block polymer available as Kraton 603 from Shell Chemical. The loop tape was slit into patches 50 having a width of 38 mm.

The fingerlift tape 54 was prepared by first coating an 85 µm thick blue polypropylene film on one side with a solventless UV curable silicone low adhesion backsize and on the other side with a 35 µm thick layer 58 of pressure-sensitive adhesive based on a synthetic rubber available as Kraton 1161 from Shell Chemical. The fingerlift tape 54 was cut into strips having a width of 20 mm.

The 20 mm wide fingerlift tape 54 was then folded over one end of the loop tape described above in a manner such that a 10 mm wide area of the loop surface was covered with fingerlift tape 54. The remainder of the fingerlift tape 54 was folded over, partially onto itself for a width of 3 mm, and then contacting the adhesive coated layer 52 of the loop patch 50 for 4 mm to form a fingerlift 60. The distance between the covered area of the loop side of the loop patch 50 and the covered area of the PSA-coated layer 52 of the loop patch 50 (the offset distance) was 6 mm.

A release tape 42 was prepared by coating a 72 µm thick polypropylene film on one side with a solventless silicone UV curable low adhesion backing 44 and on the other side with a 27 µm thick layer 46 of pressure-sensitive adhesive based on a synthetic rubber block polymer available as Kraton 603 from Shell Chemical. The release tape 42 was slit to a width of 50 mm.

The loop patch 50 described above, bearing the fingerlift tape 54 on one edge 62 as described, was then adhered to the non-PSA coated side of the release tape 42 in such a manner that the fingerlift 36 was aligned with the edge of the release tape 42. The portion of the release tape 42 not covered by loop patch 50 was folded over onto itself in a backing-to-backing fashion so that the folded over area adjacent to the loop area bore exposed PSA.

The release tape/loop patch laminate thus prepared was then laminated to the fastening tape 24 in such a manner that 1) the open adhesive layer 46 of the release tape 42 adhered to the fastening tape 24 in the area adjacent to the hook patch 38, 2) the hook area and loop area mechanically engaged, 3) that the two respective fingerlifts 36,60 were set off by about 4 mm.

The completed laminate having an overall width of 64 mm was then wound into a planetary roll.

Sections of the laminate having a length of 40 mm were then cut from the roll forming the multiple layer connection tape 22 and applied to the back ears 17 of disposable diapers 10.

## Claims

1. A connection tape (22) for the fastening of two articles or for the fastening of a first part (15) of an article to the second part (16) of the article, comprising:
- a fastening tape (24) having a first section (32) adapted to be fixed to the first part (15) of the article and having a second section (34) provided with a first fastener member (38) of a two-part mechanical fastener,
- a target tape (26) having a first surface adapted to be fixed on the second part (16) of the article and having a second surface provided with a second fastener member (50) of said two-part mechanical fastener,
- wherein said first and second fastener members (38,50) are capable to be mechanically engaged with and mechanically disengaged from each other and
- wherein said second surface of said target tape (26) is provided with a first end section within which said first member (38) cannot be engaged with said second member (50); said first end section of said second surface of said target tape (26) being distal from said first section (32) of said fastening tape (24) when said first and second fastener members (38,50) are mechanically engaged.

2. A connection tape according to claim 1, wherein said second fastener member (50) extends into said first end section of said second surface of said target tape (26) and, within said first end section, is covered by a cover tape (54) having an adhesive surface (58) adhered to said second member (50).

3. A connection tape according to claim 1 or 2, wherein an end area of said first surface of the target tape (26) distal from said first section (32) of said fastening tape (24) when said first and second fastener members (38,50) are mechanically engaged, is prevented from being fixed to the second article or the second part of the article said first end section of said second surface of said target tape (26) is wider than said end area of said first surface of said target tape (26).

4. A connection tape according to claim 2, wherein said cover tape (54) extends from said first end section of said second surface to said end area of said first surface of the target tape (26) and is adhered thereto so as to provide a fingerlift (60).

5. A connection tape according to claim 1, wherein said first and second members (38,50) are provided with mechanically engagable and disengagable fastening elements (40,51) projecting from said first and second members (38,50) and wherein said second member (50) within said first end section of said target tape (26) is free of fastening elements.

6. A connection tape according to any one of claims 1 to 5, wherein said fastening tape (24) is provided with one adhesive surface (30) on which the first fastener member (38) is mounted and wherein an end portion of said second section (34) of said fastening tape (24) distal from its first section (32) is folded back so as to build a fingerlift (36).

7. A connection tape according to any one of claims 1 to 6, wherein a release tape (42) extends from said fastening tape (24), said release tape (42) has an adhesive surface (46) and an opposite release surface (44), wherein the fastening tape (24) is provided with an adhesive surface (30), said fastening and release tape (24,42) forming a Y-shaped structure with the release tape (42) and the first section (32) of the fastening tape (24) adapted to be adhered to in particular opposite sides of the first part (16) of the article.

8. A connection tape according to claim 7, wherein said first surface of said target tape (26) comprises an adhesive surface (30) for releasably adhering to said release surface (44) of said release tape (42).

9. A connection tape according to claim 7 or 8, wherein said release tape (42) and said fastening tape (24) are build as one contiguous tape with one adhesive surface, said release tape (42) in a middle section forming said second section of the fastening tape (24) is folded back so as to form the Y-shaped structure.

10. Connection tape (22) according to any of claims 1 - 9, said connection tape (22) being planetary wound as a coil or level wound onto a core (76, 78) to provide a roll.

11. Fastening system for fastening two parts (15, 16) of an article to each other, in particular for fastening of an absorbent article, for example a disposable diaper (10), said fastening system comprising a connection tape (22) according to any of claims 1-9.

12. Absorbent article, in particular a disposable diaper, comprising: a layer (11) of absorbent material for absorbing liquid of a human body, an outer layer (13) covering the absorbent layer (11) at its outer surfaces (11,13), said assembly of layers having opposite first and second ends (15, 16) and a fastening system according to claim 11 for connecting said parts (15, 16) of the assembly of layers (11,13) with each other so as to fasten said assembly of layers (11,13) on a human body, wherein the fastening tape (24) of the connection tape (22) in its first section (32) being fixed to said first end (15) and the target tape (26) of the connection tape (22) being adapted to be fixed to the second end (16) of the assembly of layers (11,13).

## Patentansprüche

1. Verbindungsband (22) zum Befestigen von zwei Gegenständen oder zum Befestigen eines ersten Teils (15) eines Gegenstandes am zweiten Teil (16) eines Gegenstandes, das Folgendes aufweist:
- ein Befestigungsband (24) mit einem ersten Abschnitt (32), welcher zum Befestigtwerden am ersten Teil (15) des Gegenstandes angepasst ist, sowie mit einem zweiten Abschnitt (34), welcher mit einem ersten Befestigungselement (38) eines mechanischen Zwei-Teile-Befestigungsmittels ausgestattet ist,
- ein Zielband (26) mit einer ersten Oberfläche, welches zum Befestigtwerden am zweiten Teil (16) des Gegenstandes angepasst ist, sowie mit einer zweiten Oberfläche, welche mit einem zweiten Befestigungselement (50) des mechanischen Zwei-Teile-Befestigungsmittels ausgestattet ist,
- wobei das erste und das zweite Befestigungselement (38, 50) geeignet sind, mechanisch miteinander verbunden und mechanisch voneinander gelöst zu werden, und
- wobei die zweite Oberfläche des Zielbandes (26) mit einem ersten Endabschnitt ausgestattet ist, in dem das erste Element (38) nicht am zweiten Element (50) befestigt werden kann, wobei der erste Endabschnitt der zweiten Oberfläche des Zielbandes (26) distal vom ersten Abschnitt (32) des Befestigungsbandes (24) liegt, wenn das erste und das zweite Befestigungselement (38, 50) mechanisch miteinander verbunden sind.

2. Verbindungsband nach Anspruch 1, wobei sich das zweite Befestigungselement (50) in den ersten Endabschnitt der zweiten Oberfläche des Zielbandes (26) hinein erstreckt und innerhalb des ersten Endabschnitts durch ein Abdeckband (54) mit einer haftenden Oberfläche (58), die am zweiten Element (50) angeheftet ist, abgedeckt ist.

3. Verbindungsband nach Anspruch 1 oder 2, wobei ein Endbereich der ersten Oberfläche des Zielbandes (26), der distal vom ersten Abschnitt (32) des Befestigungsbandes (24) liegt, wenn das erste und das zweite Befestigungselement (38, 50) mechanisch miteinander verbunden sind, daran gehindert wird, am zweiten Gegenstand oder am zweiten Teil des Gegenstandes befestigt zu werden, wobei der erste Endabschnitt der zweiten Oberfläche des Zielbandes (26) breiter als der Endbereich der ersten Oberfläche des Zielbandes (26) ist.

4. Verbindungsband nach Anspruch 2, wobei sich das Abdeckband (54) vom ersten Endabschnitt der zweiten Oberfläche zum Endbereich der ersten Oberfläche des Zielbandes (26) erstreckt und an diese angeheftet ist, so dass ein Anfasser (60) bereitgestellt wird.

5. Verbindungsband nach Anspruch 1, wobei das erste und das zweite Element (38, 50) mit mechanisch verbindbaren und lösbaren Befestigungskomponenten (40, 51) ausgestattet sind, die vom ersten und zweiten Element (38, 50) hervorstehen, und wobei das zweite Element (50) im ersten Endabschnitt des Zielbandes (26) frei von Befestigungskomponenten ist.

6. Verbindungsband nach einem der Ansprüche 1 bis 5, wobei das Befestigungsband (24) mit einer haftenden Oberfläche (30) ausgestattet ist, auf der das erste Befestigungselement (38) angebracht ist, und wobei ein Endabschnitt des zweiten Abschnitts (34) des Befestigungsbandes (24), der distal von seinem ersten Abschnitt (32) liegt, zurückgefaltet ist, so dass ein Anfasser (36) gebildet wird.

7. Verbindungsband nach einem der Ansprüche 1 bis 6, wobei sich vom Befestigungsband (24) ein Trennband (42) erstreckt, wobei das Trennband (42) eine haftende Oberfläche (46) und eine gegenüberliegende Trennoberfläche (44) aufweist, wobei das Befestigungsband (24) mit einer haftenden Oberfläche (30) ausgestattet ist, wobei das Befestigungs- und das Trennband (24, 42) eine Y-förmige Struktur mit dem Trennband (42) bilden und der erste Abschnitt (32) des Befestigungsbandes (24) dafür angepasst ist, an bestimmten gegenüberliegenden Seiten des ersten Teils (16) des Gegenstandes angeheftet zu werden.

8. Verbindungsband nach Anspruch 7, wobei die erste Oberfläche des Zielbandes (26) eine haftende Oberfläche (30) zum lösbaren Anheften an die Trennoberfläche (44) des Trennbandes (42) umfasst.

9. Verbindungsband nach Anspruch 7 oder 8, wobei das Trennband (42) und das Befestigungsband (24) als ein zusammenhängendes Band mit einer haftenden Oberfläche gebildet sind, wobei das Trennband (42) in einem Mittelbereich, der den zweiten Abschnitt des Befestigungsbandes (24) bildet, zurückgefaltet ist, so dass eine Y-förmige Struktur gebildet wird.

10. Verbindungsband (22) nach einem der Ansprüche 1 bis 9, wobei das Verbindungsband (22) planetarisch als Spirale oder eben um einen Kern (76, 78) gewickelt wird, um eine Rolle bereitzustellen.

11. Befestigungssystem zum Befestigen von zwei Teilen (15, 16) eines Gegenstandes aneinander, insbesondere zum Befestigen eines saugfähigen Gegenstandes, zum Beispiel einer Wegwerfwindel (10), wobei das Befestigungssystem ein Verbindungsband (22) nach einem der Ansprüche 1 - 9 aufweist.

12. Saugfähiger Gegenstand, insbesondere eine Wegwerfwindel, der Folgendes aufweist: eine Schicht (11) aus saugfähigem Material zum Aufsaugen von Flüssigkeit eines menschlichen Körpers, eine Außenschicht (13), die die saugfähige Schicht (11) an ihren Außenflächen (11, 13) abdeckt, wobei die Anordnung der Schichten gegenüberliegende erste und zweite Enden (15, 16) sowie ein Befestigungssystem nach Anspruch 11 zum Verbinden der Teile (15, 16) der Anordnung der Schichten (11, 13) miteinander aufweist, so dass die Anordnung der Schichten (11, 13) an einem menschlichen Körper befestigt werden kann, wobei das Befestigungsband (24) des Verbindungsbandes (22) in seinem ersten Abschnitt (32) am ersten Ende (15) befestigt wird und das Zielband (26) des Verbindungsbandes (22) dafür angepasst ist, am zweiten Ende (16) der Anordnung von Schichten (11, 13) befestigt zu werden.

## Revendications

1. Ruban de raccordement (22) pour la fixation de deux articles ou pour la fixation d'une première partie (15) d'un article à la deuxième partie (16) de l'article, comprenant :
- un ruban de fixation (24) comprenant une première section (32) adaptée pour être fixée à la première partie (15) de l'article et comprenant une deuxième section (34) pourvue d'un premier élément de fixation (38) d'un dispositif de fixation mécanique en deux parties,
- un ruban cible (26) comprenant une première surface adaptée pour être fixée sur la deuxième partie (16) de l'article et comprenant une deuxième surface pourvue d'un deuxième élément de fixation (50) dudit dispositif de fixation mécanique en deux parties,
- dans lequel lesdits premier et deuxième éléments de fixation (38, 50) sont capables d'être mécaniquement engagés l'un avec l'autre et mécaniquement désengagés l'un de l'autre et
- dans lequel ladite deuxième surface dudit ruban cible (26) est pourvue d'une première section d'extrémité dans laquelle ledit premier élément (38) ne peut pas être engagé avec ledit deuxième élément (50), ladite première section d'extrémité de ladite deuxième surface dudit ruban cible (26) étant distale relativement à ladite première section (32) dudit ruban de fixation (24) quand lesdits premier et deuxième éléments de fixation (38, 50) sont mécaniquement engagés.

2. Ruban de raccordement selon la revendication 1, dans lequel ledit deuxième élément de fixation (50) se prolonge dans ladite première section d'extrémité de ladite deuxième surface dudit ruban cible (26) et, dans ladite première section d'extrémité, est recouvert par un ruban de recouvrement (54) comportant une surface adhésive (58) collée sur ledit deuxième élément (50).

3. Ruban de raccordement selon la revendication 1 ou 2, dans lequel une zone d'extrémité de ladite première surface du ruban cible (26) distale relativement à ladite première section (32) dudit ruban de fixation (24), quand lesdits premier et deuxième éléments de fixation (38, 50) sont mécaniquement engagés, est empêchée de se fixer au deuxième article ou à la deuxième partie de l'article, ladite première section d'extrémité de ladite deuxième surface dudit ruban cible (26) étant plus large que ladite zone d'extrémité de ladite première surface dudit ruban cible (26).

4. Ruban de raccordement selon la revendication 2, dans lequel ledit ruban de recouvrement (54) se prolonge à partir de ladite première section d'extrémité de ladite deuxième surface jusqu'à ladite zone d'extrémité de ladite première surface du ruban cible (26) et est collé dessus de façon à former une prise de soulèvement manuel (60).

5. Ruban de raccordement selon la revendication 1, dans lequel le premier et le deuxième éléments (38, 50) sont pourvus de pièces de fixation (40, 51) capables d'être engagées et désengagées mécaniquement qui dépassent desdits premier et deuxième éléments (38, 50) et dans lequel ledit deuxième élément (50) dans ladite première section d'extrémité dudit ruban cible (26) est exempt de pièces de fixation.

6. Ruban de raccordement selon l'une quelconque des revendications 1 à 5, dans lequel ledit ruban de fixation (24) est pourvu d'une surface adhésive (30) sur laquelle le premier élément de fixation (38) est monté et dans lequel une partie d'extrémité de ladite deuxième section (34) dudit ruban de fixation (24) distale relativement à sa première section (32) est repliée de façon à former une prise de soulèvement manuel (36).

7. Ruban de raccordement selon l'une quelconque des revendications 1 à 6, dans lequel un ruban de séparation (42) se prolonge à partir dudit ruban de fixation (24), ledit ruban de séparation (42) comporte une surface adhésive (46) et une surface anti-adhésive opposée (44), dans lequel le ruban de fixation (24) est pourvu d'une surface adhésive (30), lesdits ruban de fixation et ruban de séparation (24, 42) formant une structure en forme de Y, le ruban de séparation (42) et la première section (32) du ruban de fixation (24) étant adaptés pour un collage sur des côtés en particulier opposés de la première partie (16) de l'article.

8. Ruban de raccordement selon la revendication 7, dans lequel ladite première surface dudit ruban cible (26) comprend une surface adhésive (30) pour un collage de façon amovible sur ladite surface anti-adhésive (44) dudit ruban de séparation (42).

9. Ruban de raccordement selon la revendication 7 ou 8, dans lequel ledit ruban de séparation (42) et ledit ruban de fixation (24) sont construits sous la forme d'un ruban contigu avec une surface adhésive, ledit ruban de séparation (42) dans une section intermédiaire formant ladite deuxième section du ruban de fixation (24) étant replié de façon à former la structure en forme de Y.

10. Ruban de raccordement (22) selon l'une quelconque des revendications 1 à 9, ledit ruban de raccordement (22) étant enroulé par enroulement planétaire sous la forme d'une bobine ou enroulé de niveau sur un noyau (76, 78) pour produire un rouleau.

11. Système de fixation pour la fixation de deux parties (15, 16) d'un article l'une à l'autre, en particulier pour la fixation d'un article absorbant, par exemple une couche jetable (10), ledit système de fixation comprenant un ruban de raccordement (22) selon l'une quelconque des revendications 1 à 9.

12. Article absorbant, en particulier une couche jetable, comprenant : une couche (11) de matériau absorbant pour absorber le liquide d'un corps humain, une couche externe (13) recouvrant la couche absorbante (11) sur ses surfaces externes (11, 13), ledit ensemble de couches comportant une première et une deuxième extrémités opposées (15, 16) et un système de fixation selon la revendication 11 pour le raccordement desdites parties (15, 16) de l'ensemble de couches (11, 13) l'une avec l'autre, de façon à fixer ledit ensemble de couches (11, 13) sur un corps humain, dans lequel le ruban de fixation (24) du ruban de raccordement (22), dans sa première section (32), est fixé à ladite première extrémité (15) et le ruban cible (26) du ruban de raccordement (22) est adapté pour être fixé à la deuxième extrémité (16) de l'ensemble de couches (11, 13).
